# EUROPEAN PATENT APPLICATION

(11) **EP 2 064 960 A1**
(43) Date of publication of application: **03.06.2009**
(21) Application number: 07425761.9
(22) Date of filing: 29.11.2007
(51) Int. Cl.: A23L 1/30, A23L 1/00, A23P 1/04

(54) **Food composition with purifying function**

(71) Applicant: Paolo Oddenino Paris S.R.L., 10028 Trofarello (TO) (IT)
(72) Inventor: Oddenino, Paolo, 10028 Trofarello (TO) (IT)
(74) Representative: Robba, Pierpaolo

(57) **Abstract**

Food composition containing natural products of vegetable origin to be used for reaching and/or maintaining the ideal weight, said integrator having purifying function and comprising at least powder of cherry peduncule or, as an alternative, powder of mouse-ear hawkweed.

## Description

The invention relates to food integrators containing natural products of vegetable origin.

More precisely, the invention relates to food integrators containing natural products of vegetable origin to be used for reaching and/or maintaining the ideal weight.

Since the Fifties of the 20^{th} century, researches have been carried out in order to correlate the body weight with the frequency of diseases onset and with the mortality tax; it has been found that, for instance, a body weight "increased" with respect to the statistical mean value is the main reason of early death caused by cardiovascular diseases.

A study by the World Health Organization published in 2002 confirms that the overweight appears to be the fifth of the ten main reasons of death and it associates said overweight, in the western countries, to a mean loss of about 7.5 years of life.

Therefore, the interest is clear in suitably responding to the target that an increasing number of people sets to themselves, that is to achieve and maintain their ideal weight, not only for aesthetic reasons but above all for well-being and for living for a longer time.

Various methods exist to reach the ideal weight, but the better system, which does not imply excessive sacrifices and negative repercussions on health, is based on an adequate physical activity and on a well balanced nutritional program, in case conveniently improved with food integrators.

At present, a great number of food integrators are commercially available, which can be classified according to the following main kinds on the basis of their specific function:
- thermogenic integrators, which activate the metabolism by inducing the consumption of more calories even at rest;
- lipo-blocking integrators, which limit the absorption of fats;
- gluco-blocking integrators, which limit the absorption of carbohydrates; and
- ipocaloric substitutive meals, which replace lunch or dinner by giving a limited quantity of calories.

In addition or as an alternative to the aforesaid integrators intended for slimming, it is possible to take purifying integrators, which help the restoration of the right biliary and/or hepatic and/or renal performance; if the purifying integrator is taken alone, the results in terms of loss of weight will be obviously very modest and they will be obtained on rather long time periods, because the purifying integrators are not slimming specific products but they predispose the organism to a better use of the calories.

Food integrators nowadays known generally have the drawback of being metabolised in a slow and difficult way, while most of them appear to be also not much digestible.

The Applicant has now found a composition, belonging to the category of the above-mentioned purifying food integrators, which overcomes the aforesaid drawbacks.

The composition according to the present invention therefore relates to a food integrator with purifying function.

Said composition, which helps the diuretic functions thus facilitating to reach the ideal weight, comprises at least powder of cherry peduncule or, as an alternative, powder of mouse-ear hawkweed.

More precisely, 400 mg of composition of the food integrator according to the invention contain:
- about 270 mg of powder of cherry peduncule or, as an alternative, of powder of mouse-ear hawkweed.

The additional components of said composition include excipients such as, for instance, thickening agents, emulsifiers, anti-caking agents, sweeteners and flavouring agents, commonly used in the field.

Cherry peduncule, which is rich in potassium, aids diuresis and helps the physiologic purification of the organism.

Cherry peduncule used in the composition according to the present invention is of pure vegetable origin (*Prunus Cerasus*)and it is obtained by cryocrushing the cherry peduncule itself.

Mouse-ear hawkweed helps the diuretic and draining activity, doubling the urine volume, and it therefore appears to be useful for treating water retention and overweight; furthermore, mouse-ear hawkweed has anti-inflammatory, antibiotic, choleretic, colagogic, anti-toxic, anti-haemorrhagic, astringent and anti-septic activity.

Mouse-ear hawkweed used in the composition according to the present invention is of pure vegetable origin (*Hieraceum Pilosella L*.).

Preferably, said composition comprising cherry peduncule or mouse-ear hawkweed is in the form of capsules, preferably made of gelatin.

As far as the cherry peduncule is concerned, the suggested posology provides for taking one capsule of said composition once a day, preferably in the morning after breakfast, preferably together with a glass of water.

On the other hand, as far as the mouse-ear hawkweed is concerned, the suggested posology provides for taking one capsule of said composition once every three days, preferably in the morning after breakfast, preferably together with a glass of water.

The food integrator according to the invention appears to be improving with respect to the currently existing and commercially available ones, because it provides for lower suggested posologic dosages.

Though in the present description an exemplifying posology has been indicated for the composition of the food integrator according to the invention, the Applicant has developed a 24 tests system to be dispensed to the patient in order to identify his/her energy profile and to set a personalised nutritional program, also containing the specific prescription of said composition, to be taken simultaneously or as an alternative to other compositions of the same Applicant.

Moreover, it will be evident to the person skilled in the art that the composition according to the invention could be supplied under forms different from the one herein exemplified.

## Claims

1. Food integrator containing natural products of vegetable origin to be used for reaching and/or maintaining the ideal weight comprising at least powder of cherry peduncule or, as an alternative, powder of mouse-ear hawkweed.

2. Food integrator according to claim 1, having the following composition:
- about 270 mg of powder of cherry peduncule or, as an alternative, of powder of mouse-ear hawkweed, and
- complement on 400 mg: excipients.

3. Food integrator according to claim 1 or 2, wherein said cherry peduncule used in the composition according to the present invention is of pure vegetable origin (*Prunus Cerasus*).

4. Food integrator according to claim 1 or 2, wherein said mouse-ear hawkweed used in the composition according to the present invention is of pure vegetable origin (*Hieraceum Pilosella L*.).

5. Food integrator according to claim 3, wherein said cherry peduncule is obtained by cryocrushing the cherry peduncule itself.

6. Food integrator according to anyone of the preceding claims, wherein said food integrator is in the form of capsules.

7. Food integrator according to one of the claims 1, 2, 3, 5 or 6, whose suggested posology provides for taking one capsule of said composition containing cherry peduncule once a day, preferably in the morning after breakfast, preferably together with a glass of water.

8. Food integrator according to one of the claims 1, 2, 4 or 6, whose suggested posology provides for taking one capsule of said composition containing mouse-ear hawkweed once every three days, preferably in the morning after breakfast, preferably together with a glass of water.

9. Use of the food integrator according to anyone of the preceding claims, within a personalised nutritional program.
